# EUROPEAN PATENT SPECIFICATION

(11) **EP 1 428 548 B1**
(45) Date of publication and mention of the grant of the patent: **05.11.2008**
(21) Application number: 02027684.6
(22) Date of filing: 12.12.2002
(51) Int. Cl.: A61M 27/00, A61M 25/00

(54) **Catheter for use in peritoneovenous shunt**
Katheter verwendet in peritoneovenöser Abzweigvorrichtung
Cathéter utilisable à un dispositif péritonéovéneux de dérivation pour fluide

(43) Date of publication of application: 16.06.2004
(73) Proprietor: Piolax Medical Devices, Inc., Yokohama-shi, Kanagawa-ken (JP)
(72) Inventor: Takahashi, Hiroshi, Piolax Medical Devices Inc., Yokohama-shi, Kanagawa-ken (JP); Arai, Yasuaki, Nagoya 464-8681 (JP); Inoue, Hiroshi, Osaka 550-0002 (JP)
(74) Representative: Grünecker, Kinkeldey, Stockmair & Schwanhäusser Anwaltssozietät

(56) References cited:
- WO-A-00/56387
- WO-A-97/20584
- GB-A- 1 370 546
- US-A- 4 261 341
- US-A- 4 657 536

## Description

### BACKGROUND OF THE INVENTION

### 1. Field of the Invention

Ascites, which is a common complication of carcinoma and cirrhosis, worsens prognosis and hastens patients' death. Ascitic fluid has origin from blood and thus should be returned to circulating blood. This invention relates to a catheter for use in a peritoneovenous shunt and in transferring fluid accumulated in a peritoneal cavity, that is, peritoneal fluid or ascitic fluid to a vascular system.

### 2. Field of the Invention

U.S. Patent No. 4,418,693 discloses a technique for passing a venous tube through subcutaneous tissue from an incision, which is made in an abdomen, to an incision, which is made in a neck, by using a tubing passer and for inserting an end thereof into a subclavian vein as an example of means for moving ascitic fluid to a vascular system.

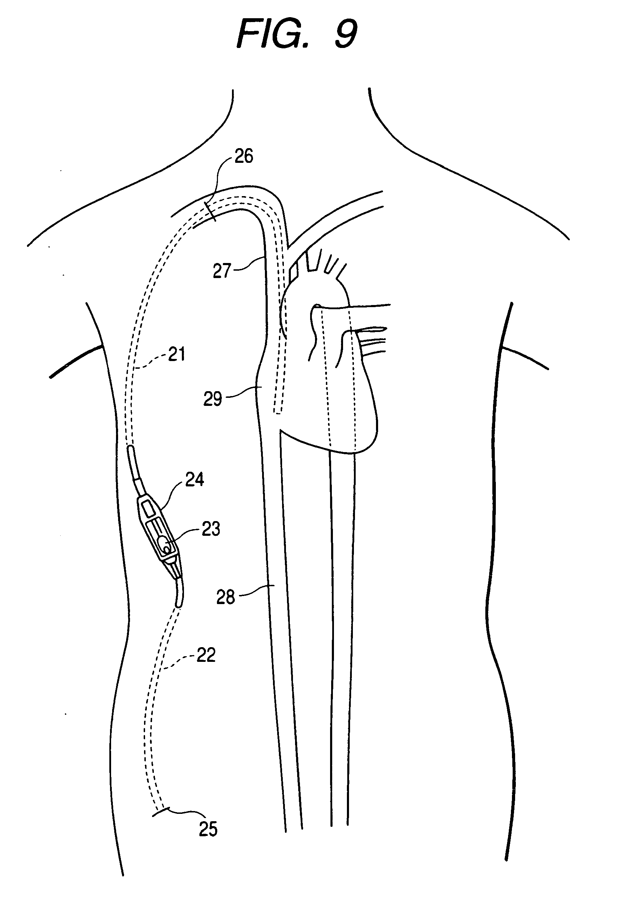

Further, a catheter for use in a peritoneovenous shunt, in which an intravenous catheter 21 and a peritoneal catheter 22 are connected to each other through a pump chamber 24 having a check valve 23, as illustrated in FIG. 9, has been known. In this case, an end portion of the intravenous catheter 21 is attached to an end of a tunneler. Then, the tunneler is inserted into a subcostal incision 25 and then pushed forward toward a subclavian incision 26 through the subcutaneous tissue. Further, the end portion of the intravenous catheter 21 is made to enter the subclavian vein 27 so that the pump chamber 24 is accommodated into a preliminary provided pump-chamber pocket, and to reach the junction of the large vein and the right atrium.

A device of the above described kind is also disclosed in WO97/20584.

However, according to the aforementioned operation method, because of the facts that a surgical region is large and that a tube or a catheter is passed through subcutenous tissue, physical load imposed on a patient is high. Moreover, economic burden put thereon is large. Additionally, the aforementioned operation method has another drawback in that patients cannot be treated at home.

### SUMMARY OF THE INVENTION

An object of the invention is to provide a catheter for use in a peritoneovenous shunt and in a novel operation method, which is relatively easy to perform, to thereby reduce the physical load and the economic burden of a patient.

The invention has been devised to achieve the foregoing object, and provides a catheter for use in a peritoneovenous shunt and in transferring peritoneal fluid accumulated in a peritoneal cavity of a patient to a vascular system. This catheter is implanted in a body of the patient in such a way as to pass right through a right atrium, a large vein, and a right hepatic vein and then penetrate a liver and reach the inside of the peritoneal cavity. This catheter comprises a peritoneal inlet provided at an end thereof and/or a peritoneal inlet provided at a side portion located to the side of the end thereof. The other end thereof is closed after implanted in the body. This catheter further comprises an outlet provided at a middle portion thereof in such a manner as to communicate with the right atrium or the large vein, and a check valve provided at the outlet or between the inlet and the outlet. In this catheter, the inlet is made to indwell within the peritoneal cavity. Further, the outlet is located in the vein.

The check valve opens when an internal pressure of the catheter is higher than an external pressure by a predetermined value, and closes when difference between the external pressure and the internal pressure is less than the predetermined value.

A first embodiment of the check valve comprises one or plural slits. Each of the slits is adapted to open when the value obtained by subtracting the external pressure from the internal pressure excesses the predetermined value.

A second embodiment of the check valve comprises one or plural liquid passage holes, which are formed in the catheter body, and one or plural flexible covers adapted to cover the holes. Both ends of each of the flexible covers are fixed to the catheter body. Further, when the value obtained by subtracting the external pressure from the internal pressure excesses the predetermined value, a middle portion of each of the flexible covers is released from a corresponding one of the liquid passage holes to thereby cause the corresponding one of the liquid passage holes to communicate with the exterior.

A third embodiment of the check valve comprises one or plural liquid passage holes, which are formed in the catheter body, and one or plural flexible covers adapted to cover the holes. An end of each of the flexible covers is fixed to the catheter body. Further, when the value obtained by subtracting the external pressure from the internal pressure excesses the predetermined value, the other end of each of the flexible covers is released from a corresponding one of the liquid passage holes to thereby cause the corresponding one of the liquid passage holes to communicate with the exterior.

A fourth embodiment of the check valve comprises an opening formed in the catheter body, and a flexible sheet that covers the opening and has one or plural slits communicating with the opening. Each of the slits is adapted to open when the value obtained by subtracting the external pressure from the internal pressure excesses the predetermined value.

A fifth embodiment of the check valve comprises one or plural liquid passage holes, which are formed in the catheter body, and a flexible cover externally provided on the catheter body in such a manner as to cover the holes and as to have one or plural slits provided at places thereon respectively corresponding to the side holes. Further, when the value obtained by subtracting the external pressure from the internal pressure excesses the predetermined value, each of the slits opens to thereby cause the corresponding one of the liquid passage holes to communicate with the exterior.

A sixth embodiment of the check valve comprises one or plural liquid passage holes, which are formed in the catheter body, and a flexible cover externally provided on the catheter body in such a manner as to cover the holes and as to have one or plural slits provided at places thereon, which do not correspond to the side holes. Further, when the value obtained by subtracting the external pressure from the internal pressure excesses the predetermined value, each of the slits opens to thereby cause the corresponding one of the liquid passage holes to communicate with the exterior.

A seventh embodiment of the check valve comprises one or plural liquid passage holes, which are formed in the catheter body, and a flexible cover that has one or plural tongue portions each adapted to cover the holes and that is externally provided on the catheter body. Further, when the value obtained by subtracting the external pressure from the internal pressure excesses the predetermined value, each of the tongue portions is released from the corresponding one of the liquid passage holes to thereby cause the corresponding one of the liquid passage holes to communicate with the exterior.

Preferably, the flexible cover or the flexible sheet is made of a material, which is softer than the catheter body or which has softness that is comparable to the catheter body.

In the case of the catheter for use in a peritoneovenous shunt, the preoperative normal length of the catheter ranges from 70 cm to 100 cm. After surgery, a 10 cm to 40 cm end portion of a right subclavian vein is ablated, so that an about 60 cm part of the right subclavian vein is left. Preferably, a silicone septum is provided in this cut end portion to thereby enable the injection of water and heparin. The normal outside diameter of the peritoneovenous shunt ranges from 2 mm to 5 mm. The normal outside diameter of the peritoneovenous shunt ranges from 2 mm to 5 mm. The inside diameter thereof ranges from 1.5 mm to 4 mm.

A peritoneal-fluid-inlet-side end portion of the catheter for use in a peritoneovenous shunt is taper-shaped or shell-shaped. A part of the catheter, which part extends from this end portion to a place located at a distance of about 20 cm therefrom, is inserted into the peritoneal cavity. A side inlet is provided at a place that is located at a distance of about 15 cm from this end portion.

A check valve is provided at a place that is located at a distance of about 35 cm to 40 cm from the peritoneal-fluid-inlet-side end portion. This valve is set in such a way as to close when the pressure of the peritoneal fluid flowing in the catheter so that the difference between this pressure and a pressure of venous blood flowing thereoutside is equal to or less than, for example, about 10 cmH₂O

The catheter for use in a peritoneovenous shunt indwells in the body for a long period. Further, the catheter is sometimes pulled out of the body so as to be replaced with new one. Thus, a material, from which a too-easy-to-deform catheter is manufactured, and a material having low tensile strength are unfavorable as the material of the catheter according to the invention. Thus, a hard-to-deform soft synthetic resin having high tensile strength, specifically, polyurethane is preferably used.

When the catheter for use in a peritoneovenous shunt according to the invention is implanted in the body, this catheter is pushed forward in an introducer. Thus, a material, which excels in pushability and smoothability, is desirable as the material of the catheter. Therefore, preferably, hydrophilization is preliminarily performed thereon. Also, preferably, heparin coating is preliminarily performed thereon so as to prevent an occurrence of blood clotting.

### BRIEF DESCRIPTION OF THE DRAWINGS

FIG. 1 is a plan view illustrating an intermediate portion of a catheter according to a first embodiment of the invention for use in a peritoneovenous shunt.
FIG. 2A is a side view illustrating an intermediate portion of a catheter according to a second embodiment of the invention for use in a peritoneovenous shunt. FIG. 2B is a plan view thereof.
FIG. 3A is a plan view illustrating an intermediate portion of a catheter according to a third embodiment of the invention for use in a peritoneovenous shunt. FIG. 3B is a longitudinally sectional view thereof.
FIG. 4 is a longitudinally sectional view illustrating a middle portion of a catheter according to a fourth embodiment of the invention for use in a peritoneovenous shunt.
FIG. 5 is a plan view illustrating a middle portion of a catheter according to a fifth embodiment of the invention for use in a peritoneovenous shunt.
FIG. 6A is a plan view illustrating an intermediate portion of a catheter according to a sixth embodiment of the invention for use in a peritoneovenous shunt. FIG. 6B is a longitudinally sectional view thereof.
FIG. 7 is a perspective view illustrating an intermediate portion of a catheter according to a seventh embodiment of the invention for use in a peritoneovenous shunt.

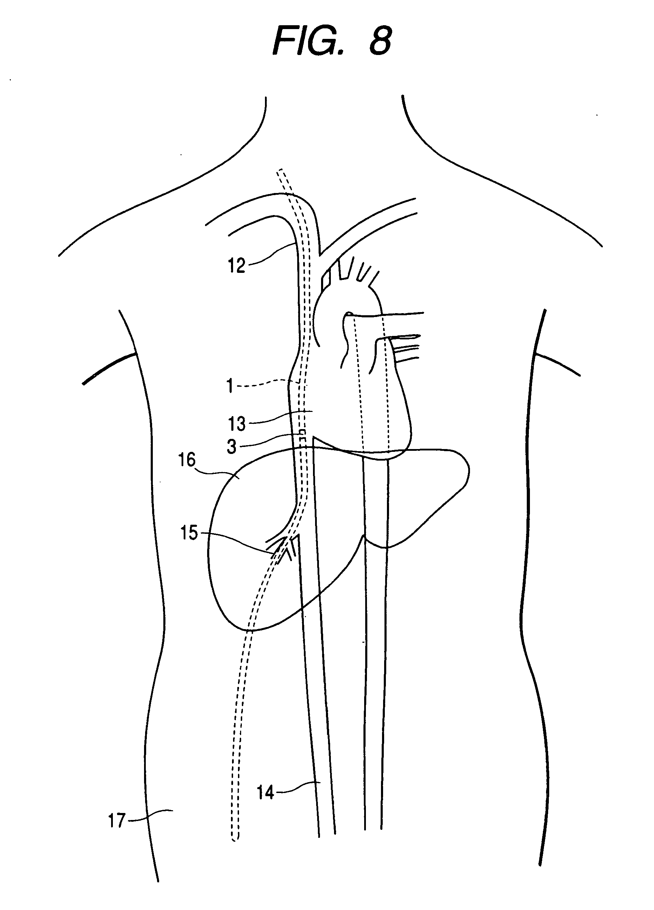

FIG. 8 is a front view illustrating a state in which the catheter according to the first embodiment of the invention is implanted in a body.
FIG. 9 is a front view illustrating a state in which a conventional venous catheter is implanted in the body.
FIG. 10 is a plan view illustrating an intermediate portion of a catheter according to an eighth embodiment of the invention for use in a peritoneovenous shunt..

### DETAILED DESCRIPTION OF THE PREFERRED EMBODIMENTS

Next, the invention is described hereinafter with reference to the accompanying drawings.

### First Embodiment

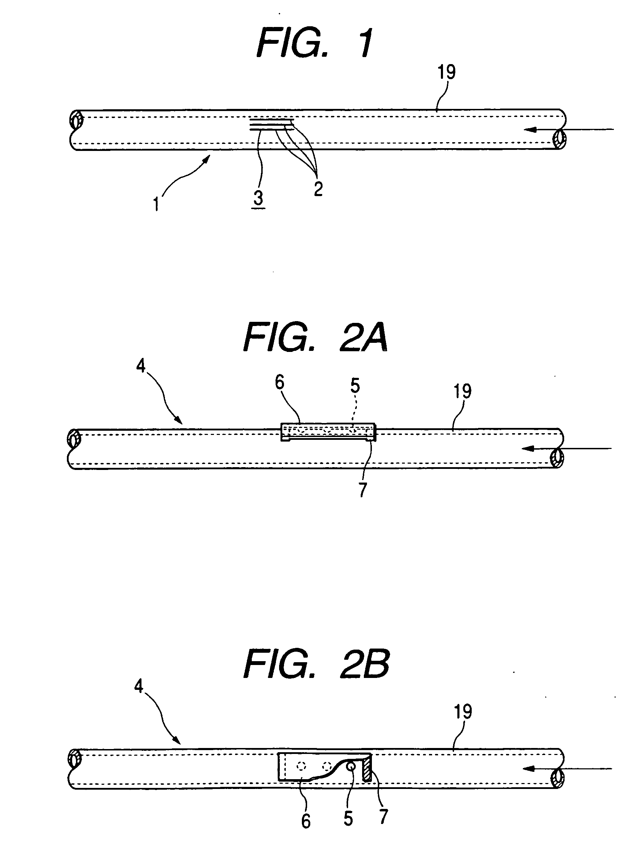

A catheter for use in a peritoneovenous shunt according to this embodiment has a tapered distal inlet at an end of a catheter body 19 formed of a polyurethane tube having 90 cm in length, 3 mm in outside diameter, and 2.8 mm in inside diameter. Further, a side inlet including a plurality of through holes is provided in a side wall at a distance of 15 cm from this end. Moreover, as shown in FIG. 1, an outlet 3 including a plurality of parallel linear slits 2 is provided at a distance of 37 cm from the distal inlet of the catheter body 19.

Each of the plurality of slits forming the outlet 3 is adapted in such a way as to open and let out fluid when the pressure of the fluid flowing in the catheter is higher than an external pressure by a predetermined value. Conversely, when the difference between the internal pressure and the external pressure is less than this predetermined value, an occurrence of back-flow can be prevented by closing the slits owing to a shape restoring force of a polyurethane tube. Thus, an occurrence of the back-flow of the fluid is prevented. Therefore, the plurality of slits of the outlet 3 also functions as a check valve.

The catheter 1 for use in a peritoneovenous shunt, which is constructed in such a manner, is pushed and goes in the introducer from the right subclavian vein 12 through the right atrium 13, the large vein 14, and the right hepatic vein 15 and then penetrates the liver 16 and reaches the inside of the peritoneal cavity 17. Thus, a part of the catheter, which extends from this end to a place located at a distance of 20 cm therefrom, is inserted into the peritoneal cavity 17. After surgery, a 10 cm right-subclavian-vein-side end portion is ablated. A silicone septum is provided in this cut end portion. The end inlet and the side inlet are made to indwell within the peritoneal cavity. Further, slits constituting the check valve are placed in the vein. This catheter 1 transfers the peritoneal fluid, which is accumulated in the peritoneal cavity of the patient, to the vascular system.

### Second Embodiment

A catheter 4 for use in a peritoneovenous shunt according to this embodiment has an outlet 5 including a plurality of liquid passage side holes in a longitudinally middle side portion, as illustrated in FIGS. 2A and 2B. The outlet 5 is covered with a rectangular flexible cover 6 formed of a polyurethane sheet. Both end portions of the flexible cover 6 are fixed to a side surface of the catheter body 19 with an adhesive agent 7.

The flexible cover 6 is formed of a flexible plastic sheet. Thus, when the pressure of fluid flowing in the catheter is higher than an external pressure by a predetermined value, so that the flexible cover 6 slightly extends and floats up from the outlet 5 to thereby open the outlet 5 and cause the fluid to flow out from a side portion of the flexible cover 6. Conversely, when the difference between the internal pressure and the external pressure is lower than the predetermined value, the flexible sheet closes the outlet 5 by covering the outlet 5 owing to the shape restoring force thereof thereby to prevent an occurrence of back-flow of the fluid. Hence, the flexible cover 6 functions as a check valve. The flexible cover 6 may be made of rubber.

The rest of the second embodiment is the same as the corresponding part of the first embodiment.

### Third Embodiment

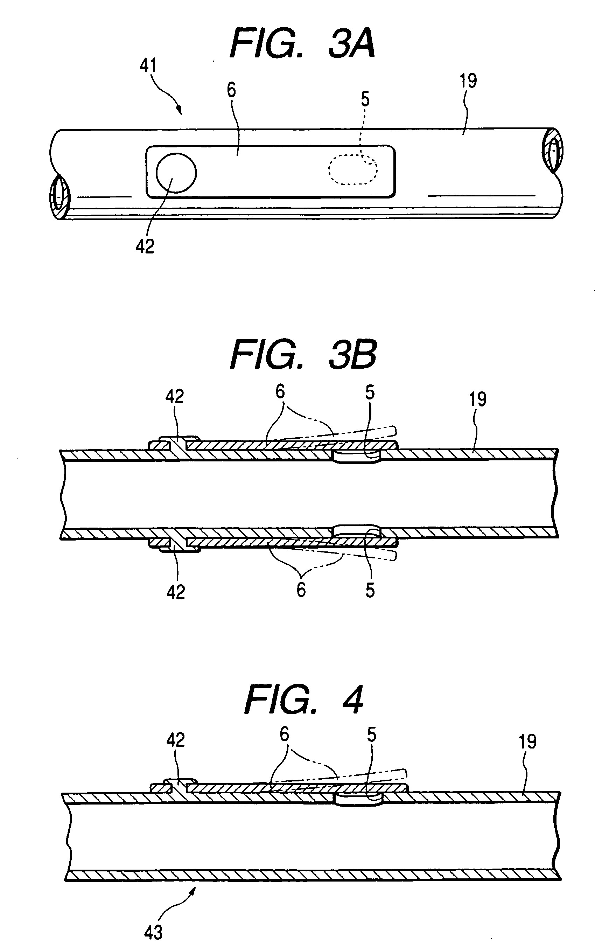

A catheter 41 for use in a peritoneovenous shunt according to this embodiment has outlets 5 each including a liquid passage side hole at a corresponding one of longitudinally middle opposed side portions of the catheter body 19, as illustrated in FIGS.3A and 3B. Each of the outlets 5 is covered with an end portion of a rectangular flexible cover 6 formed of a polyurethane sheet. A base end portion of each of the flexible covers 6 is fixed to a side surface of the catheter body 19 with a fixing portion 42.

Each of the flexible covers 6 is formed of a flexible plastic sheet. Thus, when the pressure of fluid flowing in the catheter is higher than an external pressure by a predetermined value, each of the flexible covers 6 slightly extends and floats up from the corresponding one of the outlets 5 (as indicated by two-dot chain lines in FIGS. 3B) to thereby open a corresponding one of the outlets 5 and cause the fluid to flow out from a side portion thereof. Conversely, when the difference between the internal pressure and the external pressure is lower than the predetermined value, the flexible sheet closes a corresponding one of the outlets 5 by covering the corresponding outlet 5 owing to the shape restoring force thereof thereby to prevent an occurrence of back-flow of the fluid. Hence, the flexible cover 6 functions as a check valve. The flexible cover 6 may be made of rubber.

The rest of the third embodiment is the same as the corresponding part of the first embodiment.

### Fourth Embodiment

A catheter 43 for use in a peritoneovenous shunt according to this embodiment has an outlet 5 including a liquid passage side hole in a longitudinally middle upper portion of the catheter body 19, as illustrated in FIG.4. The outlet 5 is covered with an end portion of a rectangular flexible cover 6 formed of a polyurethane sheet. A base end portion of the flexible cover 6 is fixed to a side surface of the catheter body 19 with a fixing portion 42.

The rest of the fourth embodiment is the same as the corresponding part of the third embodiment.

### Fifth Embodiment

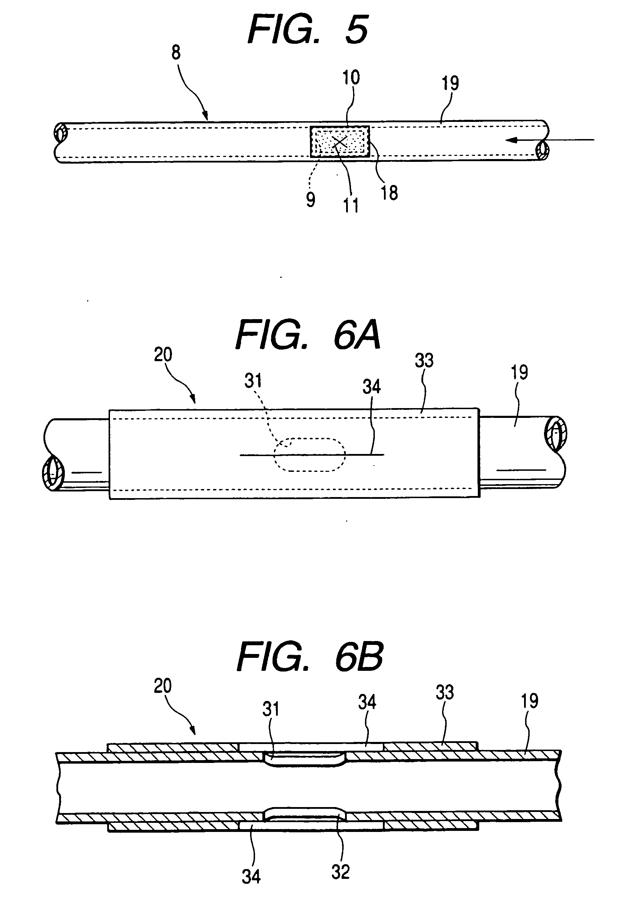

A catheter 8 for use in a peritoneovenous shunt according to this embodiment has an elliptical opening 9 in a longitudinally middle side portion as shown in FIG. 5. A flexible sheet 10, which covers this opening 9, is fixed to an edge portion of the opening of the catheter body 19 with an adhesive agent 18. An outlet 11 constituted by a cross-like slit is formed in the central portion of the flexible sheet 10.

The cross-like slit forming the outlet 11 opens against the elasticity of the flexible sheet 10 and causes the fluid to flow out therefrom when the pressure of the fluid flowing in the catheter is higher than an external pressure by a predetermined value. Conversely, when the difference between the internal pressure and the external pressure is less than the predetermined value, the cross-like slit forming the outlet 11 is closed owing to the shape restoring force thereof thereby to prevent an occurrence of back-flow of the fluid. Therefore, the outlet 11 constituted by the cross-like slit functions as a check valve. Preferably, the flexible sheet 10 is made of rubber. However, the flexible cover 6 may be formed of a flexible plastic sheet.

The rest of the fifth embodiment is the same as the corresponding part of the first embodiment.

### Sixth Embodiment

A catheter 20 for use in a peritoneovenous shunt according to this embodiment has outlets 31 and 32 each including a elliptical liquid passage side hole opened in a corresponding one of longitudinally middle opposed side portions of the catheter body 19, as illustrated in FIGS. 6A and 6B. A cylindrical flexible cover 33 is externally provided on the catheter body 19 in such a way as to cover these outlets 31 and 32. Linear slits 34 are formed in the flexible cover 33 at places respectively corresponding to the outlets 31 and 32.

Each of the slits 34 opens against the elasticity of the flexible cover 33 and causes the fluid to flow out therefrom when the pressure of the fluid flowing in the catheter is higher than an external pressure by a predetermined value. Conversely, when the difference between the internal pressure and the external pressure is less than the predetermined value, each of the slits is closed owing to the shape restoring force of the flexible cover 33 thereby to prevent an occurrence of back-flow of the fluid. Therefore, each of the slits 34 functions as a check valve. Preferably, the flexible cover 33 is made of rubber. However, the flexible cover 33 may be made of a flexible plastic material.

The rest of the sixth embodiment is the same as the corresponding part of the first embodiment.

### Seventh Embodiment

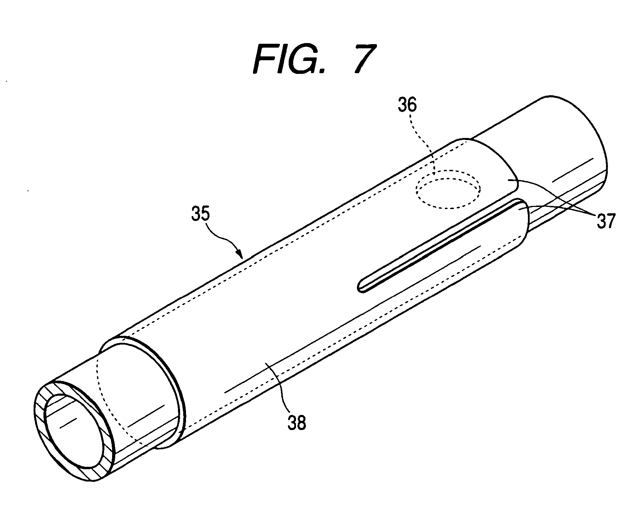

A catheter 35 for use in a peritoneovenous shunt according to this embodiment has an outlet 36, which includes a circular liquid passage side hole 36 and opened in a longitudinally middle side portion of the catheter body 19, as shown in FIG. 7. A cylindrical flexible cover 38 having a plurality of tongue portions 37, which cover this outlet 36, is externally provided on the catheter body 19.

The flexible cover 38 is formed of a flexible plastic sheet. Thus, when the pressure of fluid flowing in the catheter is higher than an external pressure by a predetermined value, the tongue portion of the flexible cover 38 floats up from the outlet 36 to thereby open the outlet 36 and cause the fluid to flow out from a side portion thereof. Conversely, when the difference between the internal pressure and the external pressure is lower than the predetermined value, the flexible cover 38 closes the outlet 36 by covering the outlet 36 owing to the shape restoring force thereof thereby to prevent an occurrence of back-flow of the fluid. Therefore, the tongue portion 37 of the flexible cover 38 functions as a check valve. The flexible cover 38 may be made of rubber.

The rest of the seventh embodiment is the same as the corresponding part of the first embodiment.

### Eighth Embodiment

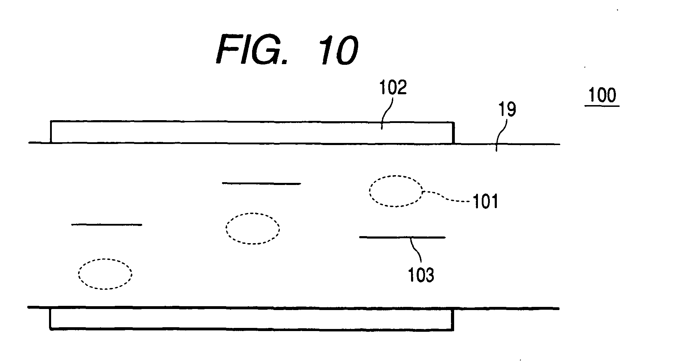

A catheter 100 for use in a peritoneovenous shunt according to this embodiment has outlets 101 each including a elliptical liquid passage side hole opened in the catheter body 19, as illustrated in FIGS.10. A cylindrical flexible cover 102 is externally provided on the catheter body 19 in such a way as to cover these outlets 101. Linear slits 103 are formed in the flexible cover 102 at places respectively not corresponding to the outlets 101.

Each of the slits 103 opens against the elasticity of the flexible cover 102 and causes the fluid to flow out therefrom when the pressure of the fluid flowing in the catheter is higher than an external pressure by a predetermined value. Conversely, when the difference between the internal pressure and the external pressure is less than the predetermined value, each of the slits is closed owing to the shape restoring force of the flexible cover 102 thereby to prevent an occurrence of back-flow of the fluid. Therefore, each of the slits 103 functions as a check valve. Preferably, the flexible cover 102 is made of rubber. However, the flexible cover 33 may be made of a flexible plastic material.

The rest of the eighth embodiment is the same as the corresponding part of the first embodiment.

As described above, the catheter for use in a peritoneovenous shunt according to the invention is implanted in the body in such a way as to pass through the right atrium, the large vein, and the right hepatic vein from the subclavian vein and then penetrate the liver and reach the inside of the peritoneal cavity. Thus, an implantation operation can easily be performed, as compared with the case of performing the conventional operation method. Consequently, the physical load and the economic burden of a patient can be reduced.

## Claims

1. A catheter (1) for use in a peritoneovenous shunt and in transferring peritoneal fluid accumulated in a peritoneal cavity of a patient to a vascular system, the catheter comprising:
an inlet provided at an end thereof and/or at a side portion located to a side of the end thereof,
the catheter (1) **characterized by** comprising:
an outlet (3) provided at a middle portion thereof; and
a check valve provided at the outlet (3) or between the inlet and the outlet,
wherein the other end thereof can be closed, and
wherein the check valve opens when an internal pressure of the catheter (1) is higher than an external pressure thereof by a predetermined value; and
wherein the check valve closes when difference between the external pressure and the internal pressure is less than the predetermined value.

2. The catheter (1) according to claim 1,
wherein the catheter (1) is capable of being implanted in a body of the patient to pass through a right atrium (13), a large vein (12), and a right hepatic vein (15) and then penetrate a liver (16) and reach inside of the peritoneal cavity (17);
wherein the other end thereof can be closed after implanted in the body;
wherein the outlet (3) is provided at the middle portion thereof to communicate with the right atrium (13) or the large vein (12);
wherein the inlet is made to indwell within the peritoneal cavity (17); and
wherein the outlet is located in the vein.

3. The catheter (1) according to at least one of the preceding claims,
wherein the catheter (1) has a length in a range of 70cm to 100cm, an outer diameter in a range of 2mm to 5mm, and an inner diameter in a range of 1.5mm to 4mm.

4. The catheter (1) according to at least one of the preceding claims,
wherein the check valve includes at least one slit (2); and
wherein the at least one slit (2) opens when the internal pressure is higher than the external pressure by the predetermined value.

5. The catheter (4) according to at least one of the claims 1-3,
wherein the check valve includes:
at least one liquid passage hole (5), which is formed in the catheter body (19); and
at least one flexible cover (6) covering the holes;
wherein both ends of the at least one flexible cover (6) are fixed to the catheter body (19); and
wherein the internal pressure is higher than the external pressure by the predetermined value, a middle portion of the at least one flexible cover is released from the at least one liquid passage hole (5) to cause the at least one liquid passage hole to communicate with the exterior.

6. The catheter according to at least one of the claims 1-3,
wherein the check valve includes:
at least one liquid passage hole (5), which is formed in the catheter body (19); and
at least one flexible cover (6) covering the at least one hole (5);
wherein one end of the at least one flexible cover (6) is fixed to the catheter body (19); and
wherein when the internal pressure is higher than the external pressure by the predetermined value, the other end of the at least one flexible cover is released from the at least one liquid passage hole (5) to cause the at least one liquid passage hole to communicate with the exterior.

7. The catheter (8) according to at least one of the claims 1-3,
wherein the check valve includes:
an opening (9) formed in the catheter body (19); and
a flexible sheet (10) that covers the opening and has at least one slit (11) communicating with the opening; and
wherein the at least one slit (11) opens when the internal pressure is higher than the external pressure by the predetermined value.

8. The catheter (20) according to at least one of the claims 1-3,
wherein the check valve includes:
at least one liquid passage holes (31), which is formed in the catheter body (19); and
a flexible cover (33) externally provided on the catheter body to cover the at least one hole (31) and to have at least one slit (34) provided at a place corresponding to the at least one hole; and
wherein when the internal pressure is higher than the external pressure by the predetermined value, the at least one slit (34) opens to cause the at least one liquid passage hole (31) to communicate with the exterior.

9. The catheter (100) according to at least one of the claims 1-3,
wherein the check valve includes:
at least one liquid passage holes (101), which is formed in the catheter body (19); and
a flexible cover (102) externally provided on the catheter body (19) to cover the at least one hole (101) and has at least one slit (103) provided at a place not corresponding to the at least one hole (101); and
wherein when the internal pressure is higher than the external pressure by the predetermined value, the at least one slit (103) opens to cause the at least one liquid passage hole (101) to communicate with the exterior.

10. The catheter according to at least one of the claims 1-3,
wherein the check valve includes:
at least one liquid passage hole (36), which is formed in the catheter body; and
a flexible cover (38) that has at least one tongue portion (37) covering the at least one hole (36) and that is externally provided on the catheter body; and
wherein when the internal pressure is higher than the external pressure by the predetermined value, the at least one tongue portion (37) is released from the at least one liquid passage hole (36) to cause the at least one liquid passage hole (36) to communicate with the exterior.

11. The catheter according to at least one of claims 5 to 10, wherein the flexible cover or the flexible sheet is made of a material, which is softer than the catheter body or which is equal to a material of the catheter body in softness.

## Patentansprüche

1. Katheter (1) zur Verwendung in einer peritoneovenösen Shunt und zum Übertragen eines in einem peritonealen Hohlraum eines Patienten angesammelten peritonealen Fluids zu einem vaskularen bzw. Gefäßsystem, wobei der Katheter umfasst:
einen Einlass, der an einem Ende hiervon und/oder an einem an einer Seite des Endes hiervon befindlichen Seitenabschnitt vorgesehen ist,
wobei der Katheter (1) **dadurch gekennzeichnet ist, dass** er umfasst:
einen Auslass (3), der an einem mittleren Abschnitt hiervon vorgesehen ist, und
ein Rückschlagventil, das an dem Auslass (3) oder zwischen dem Einlass und dem Auslass vorgesehen ist,
wobei das andere Ende hiervon geschlossen werden kann und
wobei sich das Rückschlagventil öffnet, wenn ein Innendruck des Katheters (1) um einen vorbestimmten Wert höher als ein Außendruck hiervon ist, und
wobei sich das Rückschlagventil schließt, wenn die Differenz zwischen dem Außendruck und dem Innendruck niedriger als der vorbestimmte Wert ist.

2. Katheter (1) nach Anspruch 1,
wobei der Katheter (1) dafür ausgelegt ist, in einen Körper des Patienten derart implantiert zu werden, dass er durch einen rechten Vorhof (13), eine große Vene (12) und eine rechte hepatische bzw. Lebervene (15) läuft und sodann eine Leber (16) durchdringt und das Innere des peritonealen Hohlraumes (17) erreicht,
wobei das andere Ende hiervon nach der Implantierung in den Körper geschlossen werden kann,
wobei der Auslass (3) an dem mittleren Abschnitt hiervon derart vorgesehen ist, dass er mit dem rechten Vorhof (13) oder der großen Vene (12) in Verbindung steht,
wobei der Einlass derart ausgestaltet ist, dass er im Inneren des peritonealen Hohlraumes (17) verbleibt, und
wobei der Auslass in der Vene befindlich ist.

3. Katheter (1) nach wenigstens einem der vorhergehenden Ansprüche,
wobei der Katheter (1) eine Länge in einem Bereich von 70 cm bis 100 cm, einen Außendurchmesser in einem Bereich von 2 mm bis 5 mm und einen Innendurchmesser in einem Bereich von 1,5 mm bis 4 mm aufweist.

4. Katheter (1) nach wenigstens einem der vorhergehenden Ansprüche,
wobei das Rückschlagventil wenigstens einen Schlitz (2) aufweist und
wobei sich der wenigstens eine Schlitz (2) öffnet, wenn der Innendruck um den vorbestimmtem Wert höher als der Außendruck ist.

5. Katheter (4) nach wenigstens einem der Ansprüche 1 bis 3,
wobei das Rückschlagventil beinhaltet:
wenigstens ein Flüssigkeitsdurchlassloch (5), das in dem Katheterkörper (19) ausgebildet ist, und
wenigstens eine flexible Abdeckung (6), die die Löcher abdeckt,
wobei beide Enden der wenigstens einen flexiblen Abdeckung (6) an dem Katheterkörper (19) befestigt sind und
wobei dann, wenn der Innendruck um den vorbestimmten Wert höher als der Außendruck ist, ein mittlerer Abschnitt der wenigstens einen flexiblen Abdeckung von dem wenigstens einen Flüssigkeitsdurchlassloch (5) weggenommen wird, um zu bewirken, dass das wenigstens eine Flüssigkeitsdurchlassloch mit dem Äußeren in Verbindung steht.

6. Katheter nach wenigstens einem der Ansprüche 1 bis 3,
wobei das Rückschlagventil beinhaltet:
wenigstens ein Flüssigkeitsdurchlassloch (5), das in dem Katheterkörper (19) ausgebildet ist, und
wenigstens eine flexible Abdeckung (6), die das wenigstens eine Loch (5) abdeckt,
wobei ein Ende der wenigstens einen flexiblen Abdeckung (6) an dem Katheterkörper (19) befestigt ist und
wobei dann, wenn der Innendruck um den vorbestimmten Wert höher als der Außendruck ist, das andere Ende der wenigstens einen flexiblen Abdeckung von dem wenigstens einen Flüssigkeitsdurchlassloch (5) weggenommen wird, um zu bewirken, dass das wenigstens eine Flüssigkeitsdurchlassloch mit dem Äußeren in Verbindung steht.

7. Katheter (8) nach wenigstens einem der Ansprüche 1 bis 3,
wobei das Rückschlagventil beinhaltet:
eine Öffnung (9), die in dem Katheterkörper (19) ausgebildet ist, und
eine flexible Platte (10), die die Öffnung abdeckt und wenigstens einen Schlitz (11) aufweist, der mit der Öffnung in Verbindung steht,
wobei sich der wenigstens eine Schlitz (11) öffnet, wenn der Innendruck um den vorbestimmtem Wert höher als der Außendruck ist.

8. Katheter (20) nach wenigstens einem der Ansprüche 1 bis 3,
wobei das Rückschlagventil beinhaltet:
wenigstens ein Flüssigkeitsdurchlassloch (31), das in dem Katheterkörper (19) ausgebildet ist, und
eine flexible Abdeckung (33), die außen an dem Katheterkörper derart vorgesehen ist, dass sie das wenigstens eine Loch (31) abdeckt und wenigstens einen Schlitz (34) aufweist, der an einem Ort vorgesehen ist, der dem wenigstens einen Loch entspricht,
wobei dann, wenn der Innendruck um den vorbestimmten Wert höher als der Außendruck ist, sich der wenigstens eine Schlitz (34) öffnet, um zu bewirken, dass das wenigstens eine Flüssigkeitsdurchlassloch (31) mit dem Äußeren in Verbindung steht.

9. Katheter (100) nach wenigstens einem der Ansprüche 1 bis 3,
wobei das Rückschlagventil beinhaltet:
wenigstens ein Flüssigkeitsdurchlassloch (101), das in dem Katheterkörper (19) ausgebildet ist, und
eine flexible Abdeckung (102), die außen an dem Katheterkörper (19) derart vorgesehen ist, dass sie das wenigstens eine Loch (101) abdeckt und wenigstens einen Schlitz (103) aufweist, der an einer Stelle vorgesehen ist, die nicht dem wenigstens einen Loch (101) entspricht,
wobei dann, wenn der Innendruck um den vorbestimmten Wert höher als der Außendruck ist, sich der wenigstens eine Schlitz (103) öffnet, um zu bewirken, dass das wenigstens eine Flüssigkeitsdurchlassloch (101) mit dem Äußeren in Verbindung steht.

10. Katheter nach wenigstens einem der Ansprüche 1 bis 3,
wobei das Rückschlagventil beinhaltet:
wenigstens ein Flüssigkeitsdurchlassloch (36), das in dem Katheterkörper ausgebildet ist, und
eine flexible Abdeckung (38), die wenigstens einen Zungenabschnitt (37) aufweist, der das wenigstens eine Loch (36) abdeckt, und die außen an dem Katheterkörper vorgesehen ist,
wobei dann, wenn der Innendruck um den vorbestimmten Wert höher als der Außendruck ist, der wenigstens eine Zungenabschnitt (37) von dem wenigstens einen Flüssigkeitsdurchlassloch (36) weggenommen wird, um zu bewirken, dass das wenigstens eine Flüssigkeitsdurchlassloch (36) mit dem Äußeren in Verbindung steht.

11. Katheter nach wenigstens einem der Ansprüche 5 bis 10,
wobei die flexible Abdeckung oder die flexible Platte aus einem Material bestehen, das weicher als der Katheterkörper oder mit Blick auf die Weichheit gleich einem Material des Katheterkörpers ist.

## Revendications

1. Cathéter (1) utilisable dans un dispositif péritonéoveineux de dérivation et pour transférer du fluide péritonéal accumulé dans une cavité péritonéale d'un patient vers un système vasculaire, le cathéter comprenant :
une entrée prévue à une extrémité de celui-ci et/ou au niveau d'une portion latérale située sur un côté de son extrémité,
le cathéter (1) étant **caractérisé en ce qu'**il comprend :
une sortie (3) prévue au niveau d'une portion médiane de lui-même ; et
un clapet antiretour prévu à la sortie (3) ou entre l'entrée et la sortie,
dans lequel l'autre extrémité du cathéter peut être fermée, et
dans lequel le clapet antiretour s'ouvre quand une pression interne du cathéter (1) est plus élevée qu'une pression externe de celui-ci à raison d'une valeur prédéterminée ; et
dans lequel le clapet antiretour se ferme quand une différence entre la pression externe et la pression interne est inférieure à la valeur prédéterminée.

2. Cathéter (1) selon la revendication 1,
dans lequel le cathéter (1) est capable d'être implanté dans un corps du patient pour passer à travers une oreillette droite (13), une grande veine (12) et une veine hépatique droite (15) puis pour pénétrer dans le foie (16) et atteindre l'intérieur de la cavité péritonéale (17) ;
dans lequel l'autre extrémité du cathéter peut être fermée après avoir été implantée dans le corps ;
dans lequel la sortie (3) est prévue au niveau de sa partie médiane pour communiquer avec l'oreillette droite (13) ou avec la grande veine (12) ;
dans lequel l'entrée est amenée à se loger dans la cavité péritonéale (17) ; et
dans lequel la sortie est située dans la veine.

3. Cathéter (1) selon l'une au moins des revendications précédentes,
dans lequel le cathéter (1) a une longueur dans une plage de 70 cm à 100 cm, un diamètre extérieur dans une plage de 2 mm à 5 mm, et un diamètre intérieur dans une plage de 1,5 mm à 4 mm.

4. Cathéter (1) selon l'une au moins des revendications précédentes,
dans lequel le clapet antiretour inclut au moins une fente (2) ; et
dans lequel ladite au moins une fente (2) s'ouvre quand la pression interne est plus élevée que la pression externe à raison de la valeur prédéterminée.

5. Cathéter (4) selon l'une au moins des revendications 1 à 3, dans lequel le clapet antiretour inclut :
au moins un trou de passage de liquide (5) qui est formé dans le corps de cathéter (19), et
au moins un couvercle flexible (6) qui couvre les trous ;
dans lequel les deux extrémités dudit au moins un couvercle flexible (6) sont fixées au corps de cathéter (19) ; et
dans lequel, quand la pression interne est plus élevée que la pression externe à raison de la valeur prédéterminée, une portion médiane dudit au moins un couvercle flexible est relâchée depuis ledit au moins un trou de passage de liquide (5) pour amener ledit au moins un trou de passage de liquide à communiquer avec l'extérieur.

6. Cathéter selon l'une au moins des revendications 1 à 3, dans lequel le clapet antiretour inclut :
au moins un trou de passage de liquide (5) qui est formé dans le corps de cathéter (19), et
au moins un couvercle flexible (6) qui couvre ledit au moins un trou (5) ;
dans lequel une extrémité dudit au moins un couvercle flexible (6) est fixée au corps de cathéter (19) ; et
dans lequel quand la pression interne est plus élevée que la pression externe à raison de la valeur prédéterminée, l'autre extrémité dudit au moins un couvercle flexible est relâchée depuis ledit au moins un trou de passage de liquide (5) pour amener ledit au moins un trou de passage de liquide à communiquer avec l'extérieur.

7. Cathéter (8) selon l'une au moins des revendications 1 à 3, dans lequel le clapet antiretour inclut :
une ouverture (9) formée dans le corps de cathéter (19), et
une feuille flexible (10) qui couvre l'ouverture et qui possède au moins une fente (11) communiquant avec l'ouverture ; et
dans lequel ladite au moins une fente (11) s'ouvre quand la pression interne est plus élevée que la pression externe à raison de la valeur prédéterminée.

8. Cathéter (20) selon l'une au moins des revendications 1 à 3, dans lequel le clapet antiretour inclut :
au moins un trou de passage de liquide (31) qui est formé dans le corps de cathéter (19), et
un couvercle flexible (33) prévu à l'extérieur sur le corps de cathéter pour couvrir ledit au moins un trou (31) et présentant au moins une fente (34) prévue à un emplacement correspondant audit au moins un trou ; et
dans lequel, quand la pression interne est plus élevée que la pression externe à raison de la valeur prédéterminée, ladite au moins une fente (34) s'ouvre pour amener ledit au moins un trou de passage de liquide (31) à communiquer avec l'extérieur.

9. Cathéter (100) selon l'une au moins des revendications 1 à 3, dans lequel le clapet antiretour inclut :
au moins un trou de passage de liquide (101) qui est formé dans le corps de cathéter (19), et
un couvercle flexible (102) prévu à l'extérieur sur le corps de cathéter (19) pour couvrir ledit au moins un trou (101) et possédant au moins une fente (103) prévue à un emplacement qui ne correspond pas avec ledit au moins un trou (101) ; et
dans lequel, quand la pression interne est plus élevée que la pression externe à raison de la valeur prédéterminée, ladite au moins une fente (103) s'ouvre pour amener ledit au moins un trou de passage de liquide (101) à communiquer avec l'extérieur.

10. Cathéter selon l'une au moins des revendications 1 à 3, dans lequel le clapet antiretour inclut :
au moins un trou de passage de liquide (36) qui est formé dans le corps de cathéter, et
un couvercle flexible (38) qui comporte au moins une portion (37) en forme de langue qui couvre ledit au moins un trou (36) et qui est prévue à l'extérieur sur le corps de cathéter ; et
dans lequel, quand la pression interne est plus élevée que la pression externe à raison de la valeur prédéterminée, ladite au moins une portion (37) en forme de langue est relâchée depuis ledit au moins un trou de passage de liquide (36) pour amener ledit au moins un trou de passage de liquide (36) à communiquer avec l'extérieur.

11. Cathéter selon l'une au moins des revendications 5 à 10, dans lequel le couvercle flexible ou la feuille flexible est réalisé(e) en un matériau qui est plus souple que le corps de cathéter, ou un matériau dont la souplesse est égale à celle du corps de cathéter.
